# EUROPEAN PATENT APPLICATION

(11) **EP 4 749 648 A1**
(43) Date of publication of application: **27.05.2026**
(21) Application number: 24214862.5
(22) Date of filing: 22.11.2024
(51) Int. Cl.: G16H 20/70, G16H 50/20, G16H 50/70, A61B 5/16, G06N 3/08, G06N 20/00

(54) **SYSTEM FOR PROVIDING A PLURALITY OF DIGITAL MENTAL HEALTH SERVICES**

(71) Applicant: Vasilev Kenderov, Emil, 8000 Burgas (BG)
(72) Inventor: Vasilev Kenderov, Emil, 8000 Burgas (BG); Rumenova Abadjieva, Ivelina, 1000 Sofia (BG)
(74) Representative: Germain Maureau

(57) **Abstract**

The invention relates to a system (100) designed for providing a plurality of digital mental health services to a user and/or to a mental health professional, comprising at least one module (140) among :
- a data analysis module (141) configured to interpret user data, including psychometric test results, and to provide personalized recommendations based on cognitive-behavioral therapy (CBT), analytical psychology, and art therapy;
- a support module (142) for mental health professionals that organizes and analyzes user data, generates structured reports, and aids therapeutic decision-making without providing direct diagnoses;
- a fake news detection module (143) capable of identifying media manipulation by analyzing manipulative communication techniques;
- an art therapy module (144) that enables users to engage in creative expression for purposes of personal development and stress management.

## Description

### Technical Field

The present invention relates to a system within the field of artificial intelligence, specifically designed to support mental health professionals by providing data analysis, coaching functionalities, and recommendations aimed at enhancing patient well-being. Additionally, the system functions as a personalized coaching tool, incorporating methods from cognitive-behavioral therapy (CBT), analytical psychology (inspired by Carl Jung), and art therapy to promote individual emotional well-being and personal development. These systems are chosen due to their proven efficacy and because they are corresponding with the needs of the patients/clients/users. The system is created with strict observing of the ethical norms and protection of personal data

### Technical Background

In recent years, digital applications have become essential to the daily routines of billions of people around the globe. These applications, available across various technologies, serve an expansive range of purposes, from productivity and learning to entertainment and personal development. As digital technology continues to advance, applications are evolving to offer more complex and customized experiences. They no longer just deliver static information; instead, they offer interactive features that adjust to individual needs based on user input, behaviors, and preferences, enabling a more tailored experience that aligns with personal goals and interests.

With the increasing integration of these applications into everyday life, they are also beginning to replace traditional face-to-face interactions in numerous areas, including healthcare and mental health support. This trend is expected to accelerate, and as it does, the limitations of current digital tools become more pronounced, particularly in their ability to address mental health needs effectively and continuously. Despite their widespread adoption, many applications fall short of providing the level of support necessary to improve psychological well-being and mental resilience.

For example, countless individuals receive medical or psychological diagnoses each day, which often leads to a combination of emotional, social, and financial challenges. These diagnoses can create stress and isolation, and the psychological impact may worsen physical symptoms if left unaddressed. Although traditional care options-like referrals to mental health professionals or peer support groups-can be beneficial, they frequently lack continuity. Patients may experience intense emotional struggles when alone or outside structured support environments, making it challenging to manage states of anxiety, depression, or loneliness. Over time, untreated or unidentified psychological distress can exacerbate health issues, leading to a decline in overall well-being.

Additionally, patients may not always recognize or communicate their emotional needs, which can prevent them from seeking help. Feelings of shame or discomfort related to their condition may also discourage individuals from reaching out. Consequently, gaps in traditional mental health support can significantly impact a person's quality of life and safety, highlighting a pressing need for accessible, reliable solutions that offer ongoing psychological assistance.

To address these challenges, there is a need for a more efficient and convenient system that can provide consistent mental health support and personalized guidance to individuals, along with resources for mental health professionals to monitor patient progress and organize patient data.

### Summary of the Invention

The present invention relates to a system designed for providing a plurality of digital mental health services to a user and/or to a mental health professional according to independent claim 1. Advantageous developments can be found in the dependent claims.

Thus configured, the system of the invention offers users a comprehensive set of features, including real-time analysis and feedback based on therapeutic models, such as CBT and analytical psychology, to aid in stress management and personal development.

The system of the invention further provides data-driven insights and structured analyses, helping mental health professionals in decision-making and preventing burnout.

The system of the invention further provides personalized coaching to users, encouraging self-discovery, personal growth, and emotional resilience through tools such as analytical psychology. It goes beyond typical interactive features by including a unique art therapy component, allowing users to express themselves creatively and therapeutically through activities like digital drawing and voice modulation for karaoke. This integration of art therapy encourages users to explore their emotions, fostering mental resilience and well-being in an engaging, self-paced manner.

The system of the invention also ensures compliance with relevant ethical standards by avoiding direct diagnostic or therapeutic functions while still providing valuable mental health support.

The system of the invention further includes an innovative fake news detection module that identifies and explains manipulative communication in media. This functionality helps users navigate and understand potential risks of misinformation, which can play a significant role in mental well-being.

For social connection, the system of the invention also provides an environment where individuals with similar psychological profiles can interact in a secure, ethical setting, facilitating peer support in a way that respects privacy and regulatory standards. By offering this range of interactive features, the system of the invention enhances users' ability to manage mental health challenges independently and proactively. Professional Support and Analysis: CognitAl provides data-driven insights and structured analyses, helping mental health professionals in decision-making and preventing burnout.

In conclusion, the system of the invention addresses the limitations of traditional psychological support systems by providing accessible, customizable mental health resources that support both patients and professionals. This innovative system is designed to meet the growing need for remote, personalized mental health care, enabling users to access continuous support, thereby reducing distress and improving their overall quality of life.

The system of the invention may advantageously be based on a Large Language Model (LLM) architecture capable of understanding and generating natural language text, leveraging vast amounts of data. The model's advanced natural language processing (NLP) capabilities enable it to provide customized responses, analyze large datasets, and offer tailored recommendations to both individuals and mental health professionals. Through its integration of analytical psychology, cognitive-behavioral therapy, and art therapy elements, the system of the invention delivers insights and guidance in a user-friendly way, supporting therapeutic decision-making for professionals and personal growth for individuals. Now it is 4 known, that current LLMs are indistinguishable from humans. For example. ChatGPT has been proven to have IQ 120 points. They are presented as friendly, empathetic, understanding the person, they talk with. Having information about the personality structure of patient/client, gained from psychological tests, the system of the invention will be able to make more personalized approach to the user and to provide better information to the mental health specialist, who works with the model in order to create strategy for the treatment to the patient.

### Brief Description of the Drawings

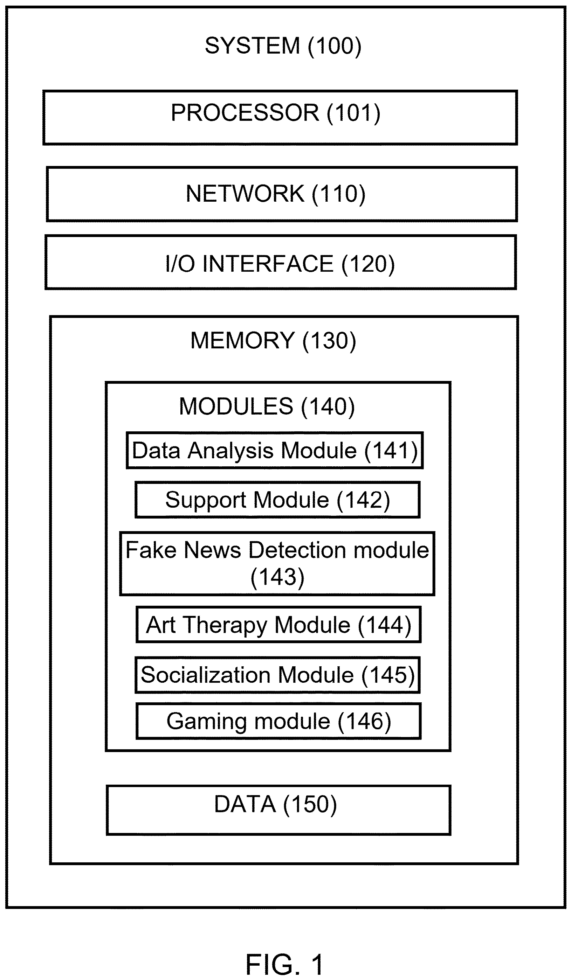

The detailed description is described with reference to the accompanying Figure 1.

FIG. 1 illustrates a block diagram showing the components of a system providing mental health services, in accordance with an embodiment of the invention.

### Detail Description

Following is an example that is illustrative only of the present invention. In particular, the invention accommodates any and every variation of the example provided below that shall serve the same purpose and is covered by the claims.

Referring to FIG. 1, the block diagram 200 illustrates key components of a system 100 designed to provide psychological health support in accordance with an embodiment of the present invention. The system 100 includes a processor 101, a memory 130 linked to the processor, and several input/output (I/O) interfaces 120. In this embodiment, the processor 101 can be implemented using various computational devices, including microprocessors, digital signal processors, or similar hardware, capable of executing program instructions stored in the memory 130. The processor 101 is responsible for managing interactions between various system modules and performing complex data analysis and decision-making tasks.

The I/O interfaces 120 provide channels for user interaction through various input and output devices. These may include input devices such as keyboards, touchscreens, microphones, cameras, and other sensors, allowing users to interact directly with the system or via user devices. On the output side, interfaces may include display screens, speakers, and other feedback mechanisms to communicate information and recommendations to users. Through these interfaces, users can provide data inputs, such as answers to mental health assessments, which are processed by the system 100 to generate tailored feedback and support.

By connecting with various devices and network types, the I/O interfaces 120 enable the system 100 to interact with a wide range of external systems, including servers and cloud storage, providing flexibility in deployment. Through wired or wireless connections, the system can support secure, real-time interactions with users and mental healthcare professionals.

The memory 130 stores multiple functional modules 140 and data 150, with memory being structured as either volatile or non-volatile types, including RAM, ROM, flash storage, and cloud-based options. The modules 140 are implemented as software routines, programs, or data structures that carry out specific tasks related to mental health support. Key modules include a data analysis module 141, a support module 142, a fake news detection module 143, an art therapy module 144, a socialization module 145, and a gaming module 146, among others. The modular structure allows the system to be flexibly implemented either on-premises or through a virtual cloud infrastructure.

The data analysis module 141 will offer the users to make psychological tests such as Minnesota Multiphasic Personality Inventory (MMPI), projective tests, level of stress, emotional intelligence. These tests will be used for granting of personalized recommendations for personal development, stress managing and motivation. For mental health specialists, the system will analyze the results of tests, providing structured data that will support decision making, without engaging in direct diagnosis. For example, the data analyis module 141 may be configured to generate sets of questions dynamically, tailored to the user's psychological profile. These questions, stored in the memory 130, cover a wide range of areas, from daily life challenges and stressors to specific mental health concerns. Based on user responses, the module adapts its questioning to further refine the insights it gathers.

The data analysis module 141 may be designed to process and interpret users' language-based inputs, whether in text or audio format, allowing the system to assess users' responses accurately. This module includes submodules for speech-to-text conversion, enabling voice inputs to be transcribed for analysis, and text-to-speech synthesis, providing users with auditory feedback. A language translation capability also allows the system to support users and professionals in multiple languages, broadening its accessibility.

The data analysis module 141 may advantageously comprise natural language processing (NLP) algorithms based on Large Language Model (LLM) trained through supervised learning techniques. Data sources include lectures, books, and anonymized records from therapeutic sessions, obtained with participant consent in strict adherence to legal and ethical standards. The data will be labeled according to key therapeutic categories, such as "motivation," "problem-solving," and "future actions planning," to allow the model to provide contextually appropriate recommendations.

The support module 142 is configured to use data gathered from the data analysis module 141 to assess the user's mental health condition and to provide tailored suggestions to mental health professionals, based on the user's specific needs. The support module 142 may thus assist mental health professionals by offering structured reports and analyses of patient data, aiding them in the decision-making process without engaging in direct diagnosis or treatment. The support module 142 is capable of offering customized advice and strategies for problem-solving, emotional well-being, and personal growth, helping professionals save time and focus on therapy by providing them with well-organized insights without making direct medical conclusions. This module will help prevent professional burnout.

The fake news detection module 143 is configured to identify media manipulation and fake news, often spread through cognitive manipulation and rhetorical abuse. It is well known, that people with specific personality disorders, more specifically those from Cluster B (psychopathy, narcissism, borderline and histrionic) are highly manipulative. By educating the LLM in the area of manipulative communication techniques (such as rhetoric abuse, cognitive manipulation) and skills, gained from mental health experts (psychologists and psychiatrists), who have experience with such patients, the fake news detection module 143 will be able to recognize the same manipulative patterns in suspicious news in the media.

The art therapy module 144 integrates tools to boost cognitive abilities and mental health through interactive entertainment. In particular, the art therapy module 144 may integrate creative tools, such as drawing software and computer graphics. Additionally, it may include a voice modulation tool, enabling users to modify their voices to become more melodic and suitable for singing, thus facilitating karaoke-based therapy or entertainment. This aspect of the training will draw on data from creative activities and artistic therapeutic sessions, where therapists' reactions and interactions with different artistic processes will be recorded and analyzed to enrich the model's understanding of creative expression in therapy.

The socialization module 145 is configured to help users to socialize with people with appropriate psychological backgrounds if this is safe for them and if they seek such socialization. In the process of socialization, the system 100 will guarantee protection of the personal data and will conform to all applicable regulations for personal inviolability. The users will be informed and will give their consent before participating in the socialization functionalities. The system 100 will apply all possible ethical standards, when there is a concurrence between the psychological profiles of certain users to prevent potentially harmful or unsuitable social interaction.

The gaming module 146 may integrate Role-Playing Games or other types of computer games (such as puzzles, strategies, etc), that will serve both for entertaining purposes, and for improving the cognitive abilities and mental health. The mental health benefits of computer games are well known and proven scientifically. In particular, computer games can increase gray matter in the brain, boost brain connectivity, and encourage developing problem solving skills, and critical thinking.

The purpose of the system 100 is to offer personalized coaching and psychological support to individuals, while assisting mental health professionals in analyzing and organizing patient data. By combining methods from CBT, Jungian psychology, and art therapy, the system of the invention fosters personal growth, stress management, and improved decision-making for professionals and users alike.

The system 100 is configured to use artificial intelligence (Al) or Machine Learning (ML) technology to provide the mental health services.

It is to be understood that the appended description is not necessarily limited to the specific features described. Rather, the specific features are disclosed as examples of implementations.

## Claims

1. A system (100) designed for providing a plurality of digital mental health services to a user and/or to a mental health professional, comprising at least one module (140) among :
- a data analysis module (141) configured to interpret user data, including psychometric test results, and to provide personalized recommendations based on cognitive-behavioral therapy (CBT), analytical psychology, and art therapy;
- a support module (142) for mental health professionals that organizes and analyzes user data, generates structured reports, and aids therapeutic decision-making without providing direct diagnoses;
- a fake news detection module (143) capable of identifying media manipulation by analyzing manipulative communication techniques;
- an art therapy module (144) that enables users to engage in creative expression for purposes of personal development and stress management.

2. The system (100) according to claim 1, wherein the data analysis module (141) comprises natural language processing (NLP) algorithms that provide real-time personalized responses and recommendations to both users and mental health professionals.

3. The system (100) according to claim 1 or claim 2, wherein the art therapy module (144) includes digital creative tools, such as drawing software, computer graphics, and voice modulation features for therapeutic activities like karaoke.

4. The system (100) according to any of claims 1 to 3, further comprising a socialization module (145) that allows users with similar psychological profiles to connect and socialize in a secure, privacy-compliant environment.

5. The system (100) according to claim 4, wherein the socialization module (145) enables users to connect with individuals with compatible psychological profiles, ensuring safe interactions through compliance with data privacy regulations and user consent protocols.

6. The system (100) according to any of claims 1 to 5, wherein the data analysis module (141) includes psychometric tests to assess stress levels, emotional intelligence, and/or other psychological traits, providing customized recommendations based on the results.

7. The system (100) according to any of claims 1 to 6, wherein the fake news detection module (143) is configured to identify cognitive manipulation and rhetorical abuse within media content as part of misinformation detection efforts.

8. The system (100) according to any of claims 1 to 7, further comprising a gaming module (146) that includes role-playing or strategy-based games designed to enhance cognitive abilities and mental well-being through interactive entertainment.

9. The system (100) according to any of claims 1 to 8, wherein the system is configured to use artificial intelligence (Al) or Machine Learning (ML) technology to provide the mental health services.

10. The system (100) according to claim 9, wherein the AI or ML technology uses Large Language Model (LLM).

11. The system (100) according to claim 10, wherein the LLM is trained using supervised learning techniques with anonymized data sourced from real psychotherapeutic sessions.

12. The system (100) according to any of claims 1 to 11, further comprising:
- a memory (130);
- a processor (101) coupled to the memory (130), wherein the processor (130) is configured to execute program instructions, stored in the memory (130), wherein the program instructions are corresponding to a plurality of modules (140), wherein the plurality of modules (140) comprise at least one module among a data analysis module (141), a support module (142), a fake news detection module (143), an art therapy module (144), a socialization module (145), and a gaming module (146);
- preferably a network (110);
- one or more I/O interface (120), wherein the one or more I/O interface (120) are communicatively coupled to the processor (101);
wherein the processor (101) is configured to execute program instructions stored in the memory (130) for:
• receiving, by the system, data (150), and
• providing mental health services to the user and/or to a mental health professional based on the data (150).
